(19) **Europäisches Patentamt**
**European Patent Office**
**Office européen des brevets**

(11) Veröffentlichungsnummer: **0 158 090**
**B1**

(12) **EUROPÄISCHE PATENTSCHRIFT**

(45) Veröffentlichungstag der Patentschrift: **29.08.90**

(51) Int. Cl.⁵: **A 61 K 31/355,** A 61 K 7/48

(21) Anmeldenummer: **85102222.8**

(22) Anmeldetag: **28.02.85**

(60) Teilanmeldung **89112798.7** eingereicht am **28/02/85.**

(54) Mittel zur Behandlung und zum Schutz der Haut.

(30) Priorität: 07.03.84 DE 3408258
23.03.84 DE 3410641
01.06.84 DE 3420459
25.07.84 DE 3427374
25.09.84 DE 3435098
15.11.84 DE 3441711
12.02.85 DE 3504695

(43) Veröffentlichungstag der Anmeldung:
16.10.85 Patentblatt 85/42

(45) Bekanntmachung des Hinweises auf die Patenterteilung:
29.08.90 Patentblatt 90/35

(84) Benannte Vertragsstaaten:
AT BE CH DE FR GB IT LI LU NL SE

(56) Entgegenhaltungen:
EP-A-0 027 987     FR-A-2 201 070
EP-A-0 133 258     FR-A-2 492 659
EP-A-0 151 987     GB-A-1 453 239
EP-A-0 151 989     US-A-3 943 248
EP-A-0 152 106     US-A-4 144 325
DE-A-2 240 187

(73) Patentinhaber: Ismail, Roshdy, Dr.
Siebengebirgs-Apotheke Siebengebirgsallee 2
D-5000 Köln 41 (Klettenberg) (DE)

(72) Erfinder: Ismail, Roshdy, Dr.
Siebengebirgs-Apotheke Siebengebirgsallee 2
D-5000 Köln 41 (Klettenberg) (DE)

(74) Vertreter: Werner, Hans-Karsten, Dr. et al
Deichmannhaus am Hauptbahnhof
D-5000 Köln 1 (DE)

(56) Entgegenhaltungen:

CHEMICAL ABSTRACTS, Band 90, Nr. 15, 9. April 1979, Seite 66, Nr. 115236g, Columbus, Ohio, US; A.S. MELKUMYAN u.a.: "Histological evaluation of the regenerative capacity of the skin following treatment of burn wounds with vitamin E ointment" & ZH. EKSP. KLIN. MED. 1978, 18(4), 52-4

CHEMICAL ABSTRACTS, Band 95, Nr. 18, 2. November 1981, Seite 365, Nr. 156366w, Columbus, Ohio, US; & JP - A - 81 75 421 (KANEBO COSMETICS, INC.) 22.06.1981

Courier Press, Leamington Spa, England.

# EP 0 158 090 B1

(56) Entgegenhaltungen:

CHEMICAL ABSTRACTS, Band 30, Nr. 21, 10.
November 1936, Spalte 7634-3, Columbus, Ohio,
US; GEZA LORANTH u.a.: "Significance of
vitamin E in dermatology" & ORVOSI HETILAP
80, 778-9 (1936)

UNLISTED DRUGS, Band 24, Nr. 1, Januar 1972,
Seite 10, Punkt o, Chatham, New Jersey, US;
UNLISTED DRUGS, Band 18, Nr. 3, März 1966,
Seite 27, Punkt k, Chatham, New Jersey, US;
ROTE LISTE, 1961, Seite 356, Editio Cantor,
Aulendorf/Württ., DE;
ROTE LISTE, 1983, Nr. 31 226, "Akne-Ex H", Nr.
31 213, "Magopsor" und Nr. 31 150, "Delta
Pimafucort", Editio Cantor, Aulendorf/Württ.,
DE;

Die Akte enthält technische Angaben, die nach
dem Eingang der Anmeldung eingereicht
wurden und die nicht in dieser Patentschrift
enthalten sind.

**Beschreibung**

Gegenstand der vorliegenden Erfindung ist ein Mittel zur Behandlung und zum Schutz der Haut unter Einsatz von Vitamin E.

Vitamin E ist bekannt als Antioxidans und Schutzvitamin für Phosphorlipide der Zellmembran. Es hält die Permeabilität und Stabilität der Zellmembran aufrecht; Lucy, Ann. N.Y. Academy of Science 203 1972, S. 4. Es ist weiterhin bekannt, daß Vitamin E eine membranabdichtende Wirkung besitzt; F. Mittelbach und G. Bodechtel, Münchner Medizinische Wochenschrift 110 (1968) 36: 1988—1993. Bei Erythrocyten, den einfachsten Zellen des menschlichen Körpers wurde festgestellt, daß Vitamin E eine Schutzwirkung für die Zellmembran darstellt. In Tierversuchen und beim Menschen wurde bewiesen, daß Anämie das erste Anzeichen von Vitamin-E-Mangel ist. Bei Gabe von hohen Vitamin-E-Dosen normalisiert sich die Hämolyse der Erythrocyten; vgl. William J. Darbey Vitamin Horm, 26 (50) S. 685—704 (1968) und Phelps DL Pediatrics 63 (6) S. 933—935 (1979). Aus diesen Literaturstellen geht hervor, daß bei oraler Verabreichung von 200 bis 800 mg Vitamin E an Patienten für einen Zeitraum von 1 bis 4 Tagen, deren Hämolyse der Erythrocyten significant verbessert wird im Vergleich zu Patienten mit Vitamin-E-Mangel.

Vitamin E ist weiterhin verwendet worden zur Behandlung der Sichelzellenanämie in einem Zeitraum von 6 bis 35 Wochen; vgl. Natt CL. Am. J. clin. 33, S. 968—971 (1980); Natt CL. Am. J. clin. nutr. 32, S. 1359—1362 (1979); Gawlik G. M. Fed. Proc. 35 (3), S. 252 (1976) und Gorash L. Bieri J. G. et al univ. Conn. Farmington, GT.

Weiterhin ist bekannt, daß 750 mg Vitamin E täglich in einem Zeitraum von 3 bis 6 Monaten erfolgreich bei Thalassamie-Patienten eingesetzt wurden, wobei eine Normalisierung der Hämolyse der Erythrocyten beobachtet wurde; vgl. Kahane I. ISR. J. med. 12 (1), S. 11—15 (1976).

Erfolgreich eingesetzt wurde Vitamin E weiterhin bei Patienten mit akuter Hepatitis und alkoholischer Hepatitis, die einen Mangel an Vitamin E im Serum haben; vgl. Yoshiakawa T. Takemura S. Kato H. et al. Japan J. Gastrovent, 74/7, S. 732—739 (1977). Schließlich wurde Vitamin E bei Patienten mit Eisenmangelanämie eingesetzt und bewirkte während eines Zeitraumes von 4 bis 8 Wochen eine Verbesserung bzw. Normalisierung des Lipid-metabolismus im Knochenmark; vgl. Takoshi Itaga, Central Clinical Laboratory Nagasaki University of Medicine, Japan.

In den deutschen Patentanmeldungen P 34 20 738, P 34 02 928, P 34 05 239, P 34 07 025, P 34 08 260, P 34 16 162, P 34 32 881, P 34 05 240, P 34 02 930, P 34 07 024, P 34 07 026, P 35 15 250, P 34 27 193 wird ferner der Einsatz von Vitamin E zur Behandlung der Venen, des Analbereichs und von Rheumaerkrankungen vorgeschlagen.

Es ist weiterhin bekannt, daß Cholesterin in menschlicher und tierischer Haut durch Ultraviolett-Licht zu Cholesterin-alpha-oxyd, einen als Krebserreger bekannten Stoff, umgewandelt wird. Versuche mit Mäusen haben gezeigt, daß bei Verabreichung von Vitamin E und C sowie zwei weiteren Antioxidantien sich kein Cholesterin-alpha-oxyd bildet (Pharm. Indu. 36, Nr. 3 (1974) Anschel, USA).

Die FR—A 22 01 070 offenbart kosmetische Zusammensetzungen mit einem Gehalt an Vitamin E-Orotat von 0,05 bis 10 Gew.-%. Diese werden zur Herstellung von Haut-Kosmetika, insbesondere Cremes, Puder und Lotionen verwendet, enthalten jedoch keinen UV-Stabilisator.

Die FR—A 24 92 659 beschreibt für die Verjüngung der Haut eingesetzte Zusammensetzungen, die Vitamin E in Mengen von 0,04 bis 0,08 Gew.-% und außerdem gegebenenfalls andere Vitamine, Emulgatoren, Fette enthalten. Auch diese Mittel enthalten keinen UV-Stabilisator.

In der GB—A 1 453 239 werden Zusammensetzungen in Form topischer Präparate beschrieben, die Vitamin E sowie ein pflanzliches Öl und geeignete Trägerstoffe, jedoch keine UV-Stabilisatoren enthalten.

Aus der US—A—4,144,325 sind Sonnenschutzmittel bekannt, die nicht resorbierbar sind und als UVabsorbierende Substanz Tocopherol und seine Derivate enthalten. In diesen Mitteln oxidiert das Tocopherol zu unwirksamen Chinonen, die ihrerseits zu Hautreizungen führen können.

Abgesehen davon, daß keines der genannten Mittel neben Vitamin E einen UV-Stabilisator enthält, liegen die Mengen an Vitamin E, die in einer Verabreichungseinheit enthalten sind, deutlich unter denen der vorliegenden Erfindung.

Es wurde non überraschenderweise gefunden, daß sich Kombinationen von Vitamin E mit UV-Stabilisatoren sowie Kombinationen von Vitamin E mit UV-Stabilisatoren und gegebenenfalls anderen Wirkstoffen als Mittel zur Behandlung und zum Schutz der Haut, insbesondere zur Behandlung von Ekzemen, Hautflechte, Hautentzündungen, Juckreiz, Allergien, Faltenbildungen, Pigmentierungen der Haut sowie Wunden, eignen. Darüberhinaus können die erfindungsgemäßen Mittel als Schutz gegen ultraviolettes Licht eingesetzt werden. Die erfindungsgemäßen Mittel sind ferner als Hautschutzmittel bei Bestrahlungen, z.B. von Krebspatienten, geeignet. Dieser neue Indikationsbereich war aufgrund des bisherigen Wissenstandes nicht vorherzusehen und eröffnet ein neues breites Anwendungsfeld für Vitamin E. Die Verwendung von Vitamin E bringt auf lange Sicht eine Stabilisierung und dauernde Beseitigung der Symptome, die Wahrscheinlichkeit der Rückfälligkeit ist dadurch sehr gering.

Die Erfindung betrifft Mittel zur Behandlung und zum Schutz der menschlichen und tierischen Haut, die dadurch gekennzeichnet sind, daß sie als äußerlich anzuwendendes resorbierebares Lichtschutzmittel zur Anwendung kommen und neben üblichen Träger- und/oder Hilfsstoffen als wesentlichen Bestandteil Vitamin E als alpha-Tocopherol und/oder dessen Ester natürlicher oder synthetischer Herkunft in Mengen von 0,5 bis 20 Gew.-% pro Darreichungseinheit sowie UV-Stabilisatoren und außerdem gegebenenfalls Vitamin C, Vitamin A, Vitamine der B-Reihe, durchblutungsfördernde und/oder gefäßerweiternde Mittel, Phospholipide, ungesättigte Fettsäuren und/oder Emulgatoren enthalten.

Vitamin E kann in allen seinen alpha-Formen verwendet werden, sowohl als freies Tocopherol als auch als Ester natürlicher oder synthetischer Herkunft. Dieser Ester kann als Acetat, Succinat oder als anderer Ester verwendet werden. Für Salben, Gele und Cremes wird bevorzugt das freie Tocopherol, z.B. D,L-alpha-Tocopherol und D-alpha-Tocopherol verwendet.

Überraschenderweise wird die Wirkung von Vitamin E in Gegenwart von gefäßerweiternden und/oder durchblutungsfördernden Mitteln erheblich gesteigert in Form von Synergismen und dadurch die Behandlungszeit verkürzt.

Insbesondere in Gegenwart von durchblutungsfördernden Mitteln, wie Heparin Natrium, Extr. Hippocastani bzw. Nicotinsäurebenzylester, Arnica oder Extractum Calendulae wird die Aufnahme des Vitamin E durch die Haut verbessert. Bei Verwendung von Heparin Natrium wird die hohe Dosierung von 30.000 bis 150.000 I.E. bevorzugt.

Weitere Mittel, die die Wirkung von Vitamin E erheblich steigern und dadurch erfindungsgemäß verwendet werden können, sind durchblutungsfördernde Mittel, wie ß-Hydroxy-äthyl-rutosid, Trimethylolrutosid, Arnicae-Extract, Nicotinsäure, Nicotinsäureester und Derivate, Xantinolnicotinat und Inositolnicotinat, sowie Salicylsäure bzw. deren Ester, Dihydroergotoxin-methan-sulphohat, Dihydroergocornin-methan-sulphonat, Dihydroergocristin-methan-sulphonat, ß-Hydroxy-äthyl-salicylat, Ol. Juniperi, Ol. Pini pumilionis (Latschenkieferöl), Ol. Eucalypti, Ol. Rosmarinae, Tinct. Camphorae bzw. Kampfer, Cinnarizin, Vincamin, Pentoxyfyllin, Bamethansulfat, Bencyclanhydrogenfumarat, ß-Pyridilcarbinol, Ginkgoflavonglykoside. Es können auch weitere Derivate der durchblutungs- bzw. gefäßerweiternden Mittel verwendet werden.

Als gefäßerweiterndes Pflanzenmittel ist Extract Calendulae aus Herba Calendulae zu nennen. Die durchblutungsfördernden Mittel können ebenfalls in Retard-Form verwendet werden.

Die erfindungsgemäßen Mittel werden äußerlich in Form von Creme, Gel, Salbe oder Lotion oder Lösung ggf. zusammen mit Emulgatoren angewendet. Die Vitamin E-Konzentration beträgt dabei 0,5 bis 20 Gew.-%. Besonders bevorzugt werden 4 bis 10 Gew.-%. Man kann auch andere Darreichungsformen zubereiten, z.B. Sprays, Tinkturen oder alkoholische Lösungen. Isopropanol bzw. Propandiol ist ein besonders bevorzugtes Lösungsmittel, das zugleich durchblutungsfördernd wirkt.

Als übliche Salben- oder Cremegrundlagen können Eucerin cum. aqua. Ungt. Cordes, Ungt. Emulsificans, sowie andere nicht wasserlösliche Salbengrundlagen bzw. deren Gemische verwendet werden. Geeignete Salbengrundlagen sind beispielsweise Wollwachs, Vaseline DAB 8, Paraffin dünnflüssig sowie Gemische derselben. Sie können auch Emulgatoren enthalten wie Cetylstearylalkohol. Geeignet als Salbengrundlagen sind auch Unguentum alkoholum lanae aquosum mit Cetiol (Ölsäureoleylester) sowie Unguentum lanette, Cetylstearylalkohol, Cetiol DAB 8, aqua conservata.

Dem Mittel gemäß der Erfindung können vorteilhaft auch weitere Vitamine, z.B. Vitamin C, A, $B_1$, $B_2$ und $B_6$ zugesetzt werden.

Die erfindungsgemäßen Salben enthalten als Grundlagen zweckmäßig 70 bis 30 Gew.-%, Wasser vorzugsweise 60 bis 40 Gew.-%, 30 bis 5 Gew.-%, vorzugsweise 25 bis 7 Gew.-%, Cetiol (Oleyloleat), 30 bis 2 Gew.-%, vorzugsweise 25 bis 2 Gew.-% Cetyl-Stearylalkohol oder andere aliphatische Alkohole.

Man kann den Cetyl-Stearylalkohol ganz oder teilweise auch durch andere emulgierende Alkohole ersetzen, z.B. durch aliphatische Alkohole oder Wollwachsalkohol bzw. Diole, Stearinol, mit aliphatischen Säuren veresterte Monoglyceride oder ähnliche Stoffe. Man kann z.B. auch Paraffin oder Vaseline zusetzen, um die Salbe streichfähig zu machen. Auch Cetiol (Oleyloleat) kann durch andere Emulgatoren, z.B. Tween® 20 oder Tween® 80 ganz oder teilweise ersetzt werden. Eine besonders bevorzugte Kombination als Grundlage für Vitamin-E-haltige Salben von Cremes ist jedoch folgende:

30 bis 20 Gew.-% Cetyl-Stearylalkohol,
20 bis 10 Gew.-% Cetiol (oleyl oleat)
60 bis 40 Gew.-% Wasser (aqua conservata).

Es ist bekannt, daß wasserhaltige Salbengrundlagen, wie Unguentum emulsificans aquosum und Unguentum alkoholum lanae aquosum geeignet sind zur Verarbeitung von wasserlöslichen Wirkstoffen. Hier überrascht, daß wasserhaltige Salbengrundlagen, die sogar über 50% Wassergehalt aufweisen können, sehr gut zur Verarbeitung fettlöslicher Wirkstoffe wie Vitamin E geeignet sind.

Überraschenderweise bringen die erfindungsgemäßen Mittel besondere Vorteile, wenn Vitamin A zugesetzt wird. Insbesondere wird die Behandlungsdauer verkürzt. Demzufolge betrifft die vorliegende Erfindung auch neue Mittel zur Behandlung und zum Schutz der Haut, die Vitamin A zusammen mit Vitamin E, UV-Stabilisatoren und durchblutungsfördernden Mitteln enthalten. Vitamin A kann in Form von Vitamin-A-Palmitat, Vitamin-A-Acetat sowie weiterer Ester des Vitamin A und/oder ß-Carotin verwendet werden. Vitamin A soll so ausgewählt sein, daß die maximale Tagesdosis 50.000 I.E. nicht überschreitet, d.h. wenn zwei Darreichungsformen pro Tag angewendet werden, soll die Dosierung bei maximal 25.000 I.E. pro Darreichungsform liegen. Die Vitamin-A-Dosis des erfindungsgemäßen Mittels liegt zwischen 5.000 und 25.000 I.E., vorzugsweise 6.000 bis 15.000 I.E.

Die Vitamine A und E neigen insbesondere in Gegenwart von anderen Wirkstoffen im wässrigen Medium sehr stark zur Klumpenbildung. Dabei besteht Gefahr, daß die fettlöslichen wertvollen Stoffe nicht absorbiert werden. Überraschend wurde festgestellt, daß geringe Mengen Emulgator, ca. 1%, ausreichen, um die Klumpenbildung zu verhindern. Die Wirkstoffe werden leichter im wässrigen Medium dispergiert bzw. suspendiert.

Eine größere Menge Emulgator ist nicht notwendig, da meistens 1 bis 7% ausreichend sind, um die Klumpenbildung zu verhindern. Man kann auch Mengen bis zu 10% oder mehr verwenden. Dabei besteht aber die Gefahr, daß man zuviele Hilfsstoffe zugibt. Die Folge können Nebenwirkungen sein, insbesondere wenn das Medikament längere Zeit verabreicht wird.

Es können die üblichen Emulgatoren, die in den medizinischen Präparaten verwendet werden, wie Tween® 20, Cremophor, aliphatische Alkohole, partialveresterte Triglyceride. Erfindungsgemäß werden jedoch Tween® 80 und Cetiol bevorzugt. Hierbei wurde beobachtet, daß bei Zugabe von ca. 10% Emulgator die Emulgierung nicht wesentlich besser ist als bei Zusatz von 5% Emulgator.

Man kann als Emulgator auch Lecithin in einer Konzentration zwischen 1 und 13% verwenden. Damit wird die Resorption der Kombination Vitamin A+E, insbesondere aber des Vitamin E begünstigt. Zwar ist auch bei Verwendung von großen Mengen Lecithin bis zu 50% eine positive Wirkung erkennbar. Geringe Mengen des Emulgators aus Lecithin sind aber ausreichend, um die Klumpenbildung der fettlöslichen Vitamine zu verhindern.

Es ist ferner zu empfehlen, ca. 1% herkömmliche Emulgatoren wie Tween® 80 beizufügen, da sie die Mischbarkeit von Lecithin mit den beiden Vitaminen begünstigen und eine Klumpenbildung verhindern. Besonders vorteilhaft für die Resorption ist die Verwendung von ca. 1% Tween® 80 mit 1 bis 13% Lecithin. Ebenso können die herkömmlichen Emulgatoren Tween® 20, Cetiol (Ölsäureoleylester) und Cremophor verwendet werden. Als Lecithinpräparat wird das Sojalecithin bezorzugt.

Die erfindungsgemäßen Mittel sind als Schutz gegen ultraviolettes Licht geeignet. Es werden erfindungsgemäß UV-Stabilisatoren zugesetzt, die hautverträglich sowie fett- und wasserlöslich sind, z.B. Eusolex[R]. Die UV-Stabilisatoren können in einer Menge von 0,1 bis 20 Gew.-% zugesetzt werden. Mengen von 0,5 bis 10 Gew.-% werden bevorzugt.

Weitere Zusatzstoffe können Lebertran und/oder ungesättigte Fettsäuren sein, z.B. Linolsäure, Linolensäure oder Ölsäure. Anstelle der ungesättigten Fettsäuren können auch Siliconöle oder Polysilixane verwendet werden.

Insbesondere für Hautschutzmittel sind die erfindungsgemäßen Mittel in Kombination mit Phospholipiden z.B. Lecithin geeignet. Durch die Phospholipide wird das Eindringen des Vitamin E in die Haut beschleunigt und dadurch die Wirksamkeit der Vitamin-E-Präparate gesteigert.

Weiterhin können zur Behandlung von Hautentzündungen den erfindungsgemäßen Vitamin-E-Präparaten bis zu 12 Gew.-% Befexamac zugesetzt werden. Bevorzugt werden 3 bis 10 Gew.-%, jeweils bezogen auf die Darreichungsform.

Es ist bekannt, daß Bufexamac-Creme oder -salbe zur Behandlung von Hautentzündungen, Allergien, Ekzemen und Juckreizen sich eignet. Überraschenderweise wird jedoch die Behandlungsdauer in Gegenwart von Vitamin E wesentlich verkürzt und die Wahrscheinlichkeit des Rückfalls vermindert. Nach dem Abklingen der Krankheit wird bevorzugt nur mit Vitamin-E-Salbe eingerieben, um einen Rückfall vorzubeugen.

Zur Behandlung von Allergien können die erfindungsgemäßen Mittel mit antiallergischen Wirkstoffen, insbesondere Antihistaminika kombiniert werden. Der Zusatz von Vitamin E zu solchen antiallergischen Wirkstoffen beschleunigt den Heilungsprozeß.

Als antiallergische Wirkstoffe werden beispielsweise
Clemastinehydrogenfumarat
Chlorphenoxaminehydrochlorid
Dimetidinmaleat
Bamipinlactat oder -hydrochlorid oder andere Salze bzw.
Ester
Propylhexedrinehydrochlorid
Tritoqualine
Dephenhydramin
Meclozinhydrochlorid, verwendet.

Neben Vitamin E und UV-Stabilisatoren sowie gegebenenfalls einem oder mehreren der oben beschriebenen Stoffe enthalten die Mittel gemäß der Erfindung die üblichen Träger- und Hilfsstoffe, was für die äußerlichen Anwendungen von Bedeutung ist.

Die nachfolgenden Beispiele dienen der näheren Erläuterung der Erfindung:

Beispiel 1
Eine Creme enthält:
10 g D-alpha-Tocopherol-Konzentrat
2 g Eusolex[R] (8020 Merck)
ad 100 g Salbengrundlage aus
22 T Cetyl-Stearylalkohol
18 T Cetiol
60 T Wasser (aqua conservata)

Beispiel 2

    Eine Creme enthält:

       8 g D,L-alpha-Tocopherolkonzentrat

       3 g Eusolex<sup>R</sup> (232 Merck)

       ad 100 g Salbengrundlage wie Beispiel 1

Beispiel 3

    Eine Creme enthält:

       12 g Vitamin E

       1 g Eusolex<sup>R</sup> (8020 Merck)

       ad 100 Salbengrundlage wie Beispiel 1

Beispiel 4

    Eine Creme enthält:

       9,0 g Vitamin E

       0,3 g Eusolex<sup>R</sup> (8020 Merck)

       ad 100 Salbengrundlage aus

       17 T Cetyl-Stearylalkohol

       8 T Weiße Vaseline

       15 T Cetiol

       60 T Wasser (aqua conservata)

Beispiel 5

    Salbe gemäße Beispiel 1 mit dem Zusatz von 1,0 g Eusolex<sup>R</sup> (8020 Merck).

**Patentansprüche**

1. Mittel zur Behandlung und zum Schutz der menschlichen und tierischen Haut, dadurch gekennzeichnet, daß es als äußerlich anzuwendendes resorbierbares Lichtschutzmittel zur Anwendung kommt und neben üblichen Träger- und/oder Hilfsstoffen als wesentlichen Bestandteil Vitamin E als alpha-Tocopherol und/oder dessen Ester natürlicher oder synthetischer Herkunft in Mengen von 0,5 bis 20 Gew.-% pro Darreichungseinheit sowie UV-Stabilisatoren und außerdem gegebenenfalls Vitamin C, Vitamin A, Vitamine der B-Reihe, durchblutungsfördernde und/oder gefäßerweiternde Mittel, Phospholipide, ungesättigte Fettsäuren und/oder Emulgatoren enthält.

2. Mittel nach Anspruch 1, dadurch gekennzeichnet, daß es zusätzlich Antihistaminika enthält.

3. Mittel nach einem der Ansprüche 1 oder 2, dadurch gekennzeichnet, daß es als ungesättigte Fettsäuren Linolsäure, Linolensäure oder Ölsäure enthält.

4. Mittel nach einem der Ansprüche 1 bis 3, dadurch gekennzeichnet, daß es Silikonöle oder Polysiloxane enthält.

5. Mittel nach einem der Ansprüche 1 bis 4, dadurch gekennzeichnet, daß es als Durchblutungsmittel Nicotinsäurebenzylester, Arnika, Extractum Calendulae Extractum Hippocastani oder Heparin-Natrium enthält.

6. Mittel nach einem der Ansprüche 1 bis 5 zur Behandlung von Ekzemen, Hautflechte, Hautentzündungen, Juckreiz, Allergien, Pigmentierungen, Faltenbildungen, Haarausfall und Wunden.

7. Mittel nach einem der Ansprüche 1 bis 6, dadurch gekennzeichnet, daß es zusätzlich Phospholipide, bevorzugt Lecithin, enthält.

8. Mittel nach einem der Ansprüche 1 bis 7, dadurch gekennzeichnet, daß es zusätzlich bis zu 12 Gew.-%, vorzugsweise 3 bis 10 Gew.-%, Befexamac, jeweils bezogen auf die Darreichungsform, enthält.

**Revendications**

1. Produit pour le traitement et la protection de la peau humaine et animale, caractérisé en ce qu'il est utilisable comme moyen de protection contre la lumière, résorbable, à usage externe et contient, à côté de substances usuelles de support ou auxiliaires, comme partie intégrante, une vitamine E comme l'alpha-Tocophérol ou un de ses esters d'origine naturelle ou synthétique, en proportion de 0,5 à 20% en poids par unité de présentation, ainsi que des stabilisateurs UV et en outre éventuellement de la vitamine C, de la vitamine A, des vitamines du groupe B, des produits accélérant la circulation sanguine ou vaso-dilatateurs, des phospholipides, des acides gras insaturés, et/ou des émulsifiants.

2. Produit selon la revendication 1, caractérisé en ce qu'il contient en outre un antihistaminique.

3. Produit selon l'une des revendications 1 ou 2, caractérisé en ce qu'il contient, comme acides gras insaturés, de l'acide linoléïque, de l'acide linolénique, ou de l'acide oléique.

4. Produit selon l'une des revendications 1 à 3, caractérisé en ce qu'il contient des huiles silicones ou des polysiloxanes.

5. Produit selon l'une des revendications 1 à 4, caractérisé en ce qu'il contient, comme produit pour la circulation sanguine, du nicotinate de benzyle, de l'arnica, un extrait de Calendula, un extrait d'Hippocastanum ou de l'héparine sodée.

6. Produit selon l'une des revendications 1 à 5, destiné au traitement des eczémas, dartres, inflammations, prurits, allergies, pigmentations, formation de rides, chute de cheveux et blessures.

7. Produit selon l'une des revendications 1 à 6, caractérisé en ce qu'il contient en outre un phospholipide, de préférence de la lécithine.

8. Produit selon l'une des revendications 1 à 7, caractérisé en ce qu'il contient en plus jusqu'à 12% en poids, de préférence 3 à 10%, chaque fois rapportés à la forme de présentation, de bufexamac.

**Claims**

1. A medicament for the treatment and protection of the human and animal skin, characterized by being applied as a resorbable light stabilizer for external application and containing as substantial ingredient vitamin E in form of alpha-Tocopherol and/or its ester(s) of natural or synthetic origin in amounts of 0.5 to 20% by weight per administration unit in addition to usual support and/or supplementary agents as well as UV absorbers and furthermore eventually vitamin C, vitamin A, vitamins of the group of the B-family, agents for stimulating blood circulation and/or vasodilator agents, phospholipids, unsaturated fatty acids and/or emulsifiers.

2. Medicament according to claim 1, characterized in that it contains additionally antihistaminic drugs.

3. Medicament according to anyone of the claims 1 or 2, characterized in that it contains linoleic acid, linolenic acid or oleic acid as unsaturated fatty acids.

4. Medicament according to anyone of the claims 1 to 3, characterized in that it contains silicone oils or polymeric siloxanes.

5. Medicament according to anyone of the claims 1 to 4, characterized in that it contains nicotinic acid, benzyl ester, arnica, extractum calendulae, extractum hippocastani or heparin-sodium.

6. Medicament according to anyone of the claims 1 to 5 for the treatment of eczema, tinea of skin, inflamation of skin, pruritus, allergy, pigmentation, formation of wrinkles, alopecia, and wounds.

7. Medicament according to anyone of the claims 1 to 6, characterized in that it contains additionally phospholipids, preferred lecithin.

8. Medicament according to anyone of the claims 1 to 7, characterized in that it contains additionally bufexamac up to 12% by weight, preferably 3 to 10% by weight related to the administration form respectively.